# EUROPEAN PATENT APPLICATION

(11) **EP 1 887 007 A2**
(43) Date of publication of application: **13.02.2008**
(21) Application number: 07119970.7
(22) Date of filing: 06.05.2004
(51) Int. Cl.: C07D 471/04, C07D 513/04, A61K 31/437, A61P 11/00, A61P 19/00

(54) **Novel compounds**

(30) Priority: 09.05.2003 SE 0301373
(62) Divisional of application: 04731525.4
(71) Applicant: AstraZeneca AB, 151 85 Södertälje (SE)
(72) Inventor: Klingstedt, Tomas, 221 87, Lund (SE); Hemmerling, Martin, 221 87, Lund (SE)

(57) **Abstract**

The present invention relates to imidazo and thiazolopyridine compounds of formula (I) which are kinase inhibitors, methods for their preparation intermediates and pharmaceutical compositions comprising them.

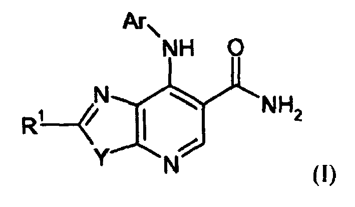

## Description

The present invention relates to novel imidazo and thiazolopyridine compounds which are JAK3 Kinase inhibitors, methods for their preparation, intermediates and pharmaceutical compositions comprising them.

Janus Kinase 3 (JAK3) is a member of the Janus family of protein kinases. Although the other members of this family are expressed by essentially all tissues, JAK3 expression is limited to hematopoetic cells. This is consistent with its essential role in signaling through the receptors for IL-2, IL-4, IL-7, IL-9, IL-13 and IL-15 by non-covalent association of JAK3 with the gamma chain common to these multichain receptors. These cytokines all have a shared function in that they are involved in lymphocyte differentiation and proliferation. XSCID patient populations have been identified with severely reduced levels of JAK3 protein or with genetic defects to the common gamma chain, suggesting that immunosupression should result from blocking signaling through the JAK3 pathway. Animal studies have suggested that JAK3 not only play a critical role in B- and T-lymphocyte maturation, but that JAK3 is constitutively required to maintain T-cell function. Modulation of immune activity through this novel mechanism can prove useful in the treatment of T-cell proliferative disorders such as transplant rejection and autoimmune diseases.

The role of JAK3 in mast cells has been described in knockout mice. Thus, IgE/antigen induced degranulation and mediator release were substantially reduced in mast cells generated from JAK3 deficient mice. JAK3 deficiency does not affect mast cell proliferation in vitro, it has also been shown that IgE receptor levels and mediator contents are identical in JAK3-/- and JAK3 +/+ mast cells. Therefore, JAK3 appears essential for the complete response of IgE challenged mast cells. The role of JAK3 in mast cell activation has been well established in murine system, however, there is no published data on mast cell function in the AR-SCID patients. Targeting JAK3 provides the basis for new and effective treatment of mast cell mediated allergic reactions.

To date a number of JAK3 inhibitors has been disclosed, among them are quinazolines (Sudbeck, E. A. et al. Clinical Cancer Res. 5(1999)1569-82, WO 00/0202) and pyrrolo[2,3-d]pyrimidines (Blumenkopf, T. A. et al. WO 99/65909).

In the current application compounds, 4-anilinoquinoline-3-carboxamides, are claimed as JAK3 inhibitors. Structurally related compounds have previously been described as kinase inhibitors e.g. WO 00/18761 and WO 98/43960 disclose substituted quinoline-3-carbonitrile derivatives. In a recent publication (Boschelli, D.H. et al. J. Med. Chem. 44(2001)822-33) one compound of the present invention has proved not to have any inhibitory capacity towards the activity of the protein tyrosine kinase Src. JAK3 is not mentioned in any of the above literature examples.

WO 02/092571 discloses a series of quinoline derivatives for use in the treatment of a disease mediated by JAK3.

There is a need for further compounds having this activity, and therefore the present invention provides a compound of formula (I): 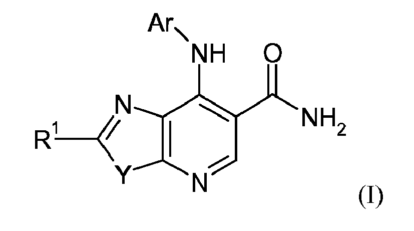 wherein:
Y is NH, S or O;
R¹ is phenyl or a 5- to 7-membered heteroaromatic ring containing 1 to 3 heteroatoms, each of which is optionally substituted by one or more groups selected from C₁-C₈ alkyl, C₁-C₈ alkoxy, S(O)ₙC₁-C₈ alkyl or a group -R²-(CH₂)ₚ-R³;
n is 0, 1 or 2;
R² is a bond, NH, NC₁-C₈ alkyl, S or O;
p is 0 - 3;
R³ is NR⁴R⁵ where R⁴ and R⁵ are independently hydrogen or C₁-C₈ alkyl, or R³ is a phenyl ring, a 5 to 7-membered saturated ring containing 1 or 2 heteroatoms selected from nitrogen, oxygen and sulphur, or a 5- to 7-membered heteroaryl group containing 1 to 3 heteroatoms selected from nitrogen oxygen and suphur, each of which can optionally substituted by one or more groups selected from CONR⁴R⁵, NR⁴R⁵, C₁-C₈ alkyl, C₁-C₈ alkoxy, S(O)ₙC₁-C₈ alkyl, hydroxyl, CN, halogen, NHCOC₁-C₈alkyl, R²-(CH₂)ₚ-OH or morpholine;
n is 1 to 4;
Ar is phenyl which can be optionally substituted by one or more groups selected from halogen, hydroxy, cyano, C₁-C₈ alkyl (itself optionally substituted by one or more hydroxy or cyano groups or fluorine atoms), CH₂-R⁶; CH₂O(CH₂)ₙOC₁₋₆ alkyl, C₁-C₈ alkyl-NR³-R⁴, or a C₂₋₆ alkyl chain optionally containing an NR⁷ group in the chain and optionally substituted by one or more OH groups and optionally terminating in a cycloalkyl or phenyl group, the cycloalkyl and phenyl groups themselves being optionally substituted by one or more hydroxyl groups;
R⁶ is a 5 to 7-membered saturated ring containing 1 or 2 heteroatoms selected from nitrogen, oxygen and sulphur, an aryl or 5- to 7-membered heteroaryl group containing 1 to 3 heteroatoms selected from nitrogen oxygen and suphur, each of which can optionally substituted by one or more substituents selected from hydroxyl or hydroxymethyl;
R⁷ is hydrogen or C₁₋₆ alkyl;
and pharmaceutically acceptable salts thereof.

The term alkyl, whether used alone or as part of another group such as alkoxy, means any straight or branched chained alkyl group. The term aryl includes phenyl and naphthyl groups. Compounds of the present invention include all stereoisomers, pure and mixed racemates, and mixtures thereof. Tautomers of compounds of formula (I) also form an aspect of the invention.

Preferably Y is NH or S.

Preferably R¹ is by SCH₃, SOCH₃, O-Ph-CONH₂, O(CH₂)₃-N(CH₃)NH(CH₂)₃-morpholine, O(CH₂)₂-morpholine, OCH₃, O(CH₂)₂-N(CH₃)₂, 2-(3-aminophenoxy), 4-methoxyphenoxy, or pyridyl or phenyl optionally substituted by OH, N(CH₃)₂, OCH₃, CN, fluoro, chloro, S(O)₂CH₃, NHCOCH₃, O(CH₂)₂OH, N(CH₃)(CH₂)₂OH, morpholine or O(CH₂)₂-OH

When R² is a 5 to 7-membered saturated ring containing 1 or 2 heteroatoms selected from nitrogen, oxygen and sulphur suitable examples include morpholine, thiomorpholine, azetidine, imidazolidine, pyrrolidine, piperidine and piperazine.

When R² is a 5- to 7-membered heteroaryl group containing 1 to 3 heteroatoms selected from nitrogen oxygen and suphur, examples include thienyl, furanyl, pyrrolyl, imidazolyl, pyridyl, pyrazinyl, pyrimidyl, pyridazinyl, triazinyl, oxazolyl, thiazolyl, isoxazolyl, pyrazolyl, oxadiazolyl, thiadiazolyl, triazolyl, imidazolyl and tetrazolyl.

Preferably Ar is phenyl which can be optionally substituted by those substituent groups of the examples herein. Especially preferred substituents include one or more substituents, which can be the same or different, selected from hydroxymethyl, ethyl, [(2-hydroxypropyl)amino]methyl, [(2-hydroxy-1-methylethyl)amino]methyl, (1H-imidazol-1-ylmethyl), [(2,3-dihydroxypropyl)amino]methyl, 3-(morpholin-4-ylmethyl), {[2-cyclohexyl-1-(hydroxymethyl)ethyl]amino}methyl, {[1-benzyl-2-hydroxyethyl]amino}methyl, (3-hydroxypyrrolidin-1-yl)methyl, {[2-hydroxy-1-phenylethyl]amino}methyl, ({2-hydroxy-1-(hydroxymethyl)-2-phenylethyl)amino}methyl, {(2-hydroxycyclohexyl)amino}methyl, {(2-hydroxy-2-(4-hydroxyphenyl)-1-methylethyl]amino}methyl, 3-hydroxypiperidin-1-ylmethyl, {[1-(hydroxymethyl)-2-methylpropyl]amino}methyl, {[4-(methylsulphonyl)benzyl]amino}methyl, and {[2-(3,4-dihydroxyphenyl)-2-hydroxyethyl]amino}methyl.

Substituents can be present on any suitable position of the Ar group. More than one substituent can be present, and these can be the same or different. One or two substituent groups are preferred.

Especially preferred compounds of the invention include those exemplified herein, both in free base form and as pharmaceutically acceptable salts.

Compounds of the invention can form pharmaceutically acceptable solvates and salts. The compounds of the formula (I) can form acid addition salts with acids, such as conventional pharmaceutically acceptable acids, for example maleic, hydrochloric, hydrobromic, phosphoric, acetic, fumaric, salicylic, citric, lactic, mandelic, tartaric, trifluoroacetic and methanesulphonic acids.

The invention also provides a method of treating or preventing a disease mediated by JAK3 which comprises administering to a mammal a compound of formula (I) as defined above.

In a further aspect the invention provides a process for the preparation of a compound of formula (I) which comprises:
(a) for compounds of formula (I) where Y is NH and Ar is phenyl substituted by CH₂NR³R⁴, reaction of a compound of formula (II): 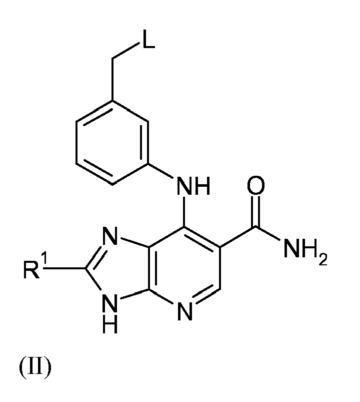 in which R¹ is as defined in formula (I) or is a protected derivative thereof and L is a leaving group, with a compound of formula (III):

   HNR³R⁴ (III)

   in which R³ and R⁴ are as defined in formula (I) or are protected derivatives thereof, or
(b) for compounds of formula (I) where Y is NH, reaction of a compound of formula (IV): 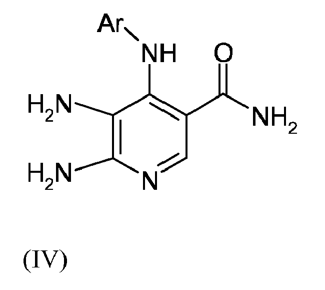 in which Ar is as defined in formula (I) or is a protected derivative thereof, with a compound of formula (V):

   R¹-CHO (V)

   in which R¹ is as defined in formula (I) or is a protected derivative thereof, or
(c) for compounds of formula (I) where Y is S and R¹ is a group -R²-(CH₂)ₚ-R³ where R² is O or NH, reaction of a compound of formula (VI): 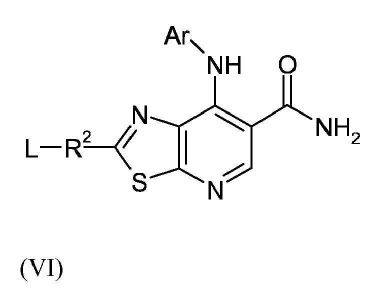 in which Ar is as defined in formula (I) or is a protected derivative thereof, R² is O or NH, and L is a leaving group, with a compound of formula (VII) or (VIII):

   R¹-OH (VII)

   R¹-NH₂ (VIII)

   in which R¹ is as defined in formula (I) or is a protected derivative thereof
   and optionally thereafter any of the above processes:
   - removing any protecting groups
   - converting a compound of formula (I) into a further compound of formula (I)
   - forming a pharmaceutically acceptable salt.

Compounds of formula (I) can be converted into futher compounds of formula (I) using standard chemistry.

Reaction of compounds (II) and (III) can be carried out in the presence of DIPA in a solvent such as NMP. Preferably the leaving group L is halogen, especially chloro. Compounds of formula (II) can be prepared by reacting compounds of formula (IX): 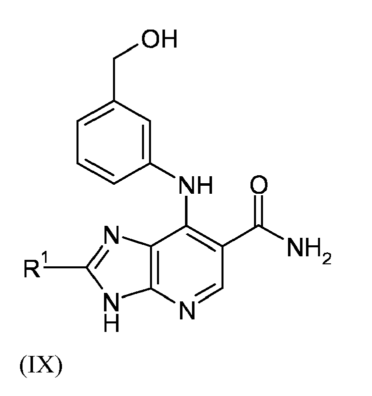 in which R¹ is as defined in formula (II) with a chlorinating agent such as SOCl₂ in dichloromethane.

Reaction of compounds of formula (IV) and (V) can be carried out in a solvent such as DMF at elevated temperature, for example at about 120°C in the presence of an oxidant such as FeCl₃. Compounds of formula (IV) can be prepared from compounds of formula (X): 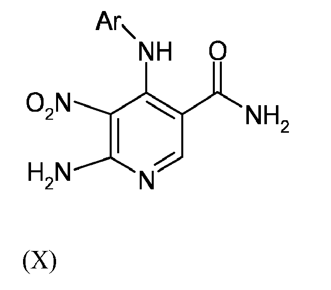 in which Ar is as defined in formula (IV) by hydrogenation using a palladium catalyst in a protic solvent such as methanol. Compounds of formula (X) can be prepared from compounds of formula (XI): 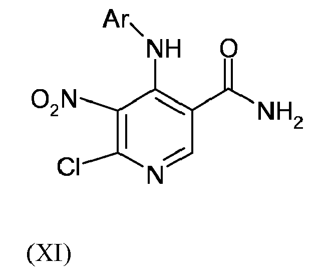 in which Ar is as defined in formula (IV) by treatment with ammonia. Compounds of formula (XI) can be prepared from compounds of formula (XII): 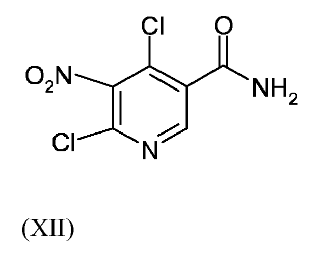 by reaction with an amine Ar-NH₂ in which Ar is as defined in formula (IV) in an aprotic solvent such as DMF at elevated temperature.

Reaction of compounds (VI) with (VII) or (VIII) can be carried out in the presence of a suitable base at ambient or elevated temperatures. Suitable leaving geoups for compounds (VI) include SO₂Me and SOMe.

Compounds of formula (VI) can be prepared by oxidation of the corresponding sulphur compound (XIII): 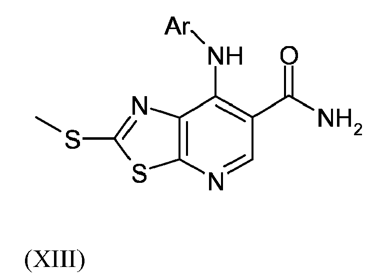 using a reagent such as 3-chloroperbenzoic acid to give the sulphoxide or potassium permanganate to give the sulphone.

Compounds of formula (XIII) are prepared from compounds of formula (XIV): 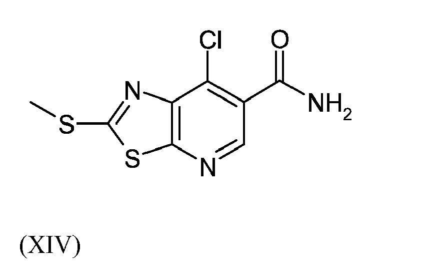 by reacting with an amine Ar-NH₂.

It will be appreciated that certain functional groups may need to be protected using standard protecting groups. The protection and deprotection of functional groups is for example, described in 'Protective Groups in Organic Chemistry', edited by J. W. F. McOmie, Plenum Press (1973), and 'Protective Groups in Organic Synthesis', 3rd edition, T. W. Greene & P. G. M. Wuts, Wiley-Interscience (1999).

Diseases mediated by JAK3 include inflammatory, immunological, and bronchopulmonary disorders.

The present invention also relates to a pharmaceutical composition for (a) treating or preventing a disorder or condition selected from organ transplant rejection, lupus, multiple sclerosis, rheumatoid arthritis, psoriasis, Type I diabetes and complications from diabetes, cancer, asthma, rhinitis, atopic dermatitis, autoimmune thyroid disorders, ulcerative colitis, Crohn's disease, Alzheimer's disease, leukemia, and other autoimmune diseases or (b) the inhibition of protein tyrosine kinases or Janus kinase 3 (JAK3) in a mammal, including a human, comprising an amount of a compound of formula I or a pharmaceutically acceptable salt thereof, effective in such disorders or conditions and a pharmaceutically acceptable carrier.

Preferably the compounds of the invention are used for the treatment of asthma, rheumatoid arthritis, and host versus graft rejection/transplantation.

The present invention also relates to a pharmaceutical composition for (a) treating or preventing a disorder or condition selected from organ transplant rejection, lupus, multiple sclerosis, rheumatoid arthritis, psoriasis, Type I diabetes and complications from diabetes, cane, asthma, rhinitis, atopic dermatitis, autoimmune thyroid disorders, ulcerative colitis, Crohn's disease, Alzheimer's disease, leukemia, and other autoimmune diseases or (b) the inhibition of protein tyrosine kinases or Janus kinase 3 (JAK3) in a mammal, including a human, comprising an amount of a compound of formula I or a pharmaceutically acceptable salt thereof, alone or in combination with a T-cell immunosuppresant or anti-inflammatory agents, effective in such disorders or conditions and a pharmaceutically acceptable carrier.

The present invention also relates to a method for the inhibition of protein tyrosine kinases or Janus Kinase 3 (JAK3) in a mammal, including human, comprising administering to said mammal an effective amount of a compound of formula I or a pharmaceutically acceptable salt thereof.

In a still further aspect the invention provides the use of a compound of formula (IA) as a therapeutic agent.

The dose of the compound to be administered will depend on the relevant indication, the age, weight and sex of the patient and may be determined by a physician. The dosage will preferably be in the range of from 0.1 mg/kg to 100 mg/kg.

The compounds may be administered topically, e.g. to the lung and/or the airways, in the form of solutions, suspensions, HFA aerosols or dry powder formulations, e.g. formulations in the inhaler device known as the Turbuhaler^{®}; or systemically, e.g. by oral administration in the form of tablets, pills, capsules, syrups, powders or granules, or by parenteral administration, e.g. in the form of sterile parenteral solutions or suspensions, or by rectal administration, e.g. in the form of suppositories.

The compounds of the invention may be administered on their own or as a pharmaceutical composition comprising the compound of the invention in combination with a pharmaceutically acceptable diluent, adjuvant or carrier. Particularly preferred are compositions not containing material capable of causing an adverse, e.g. an allergic, reaction.

Dry powder formulations and pressurized HFA aerosols of the compounds of the invention may be administered by oral or nasal inhalation. For inhalation the compound is desirably finely divided. The finely divided compound preferably has a mass median diameter of less than 10 µm, and may be suspended in a propellant mixture with the assistance of a dispersant, such as a C₈-C₂₀ fatty acid or salt thereof, (e.g. oleic acid), a bile salt, a phospholipid, an alkyl saccharide, a perfluorinated or polyethoxylated surfactant, or other pharmaceutically acceptable dispersant.

The compounds of the invention may also be administered by means of a dry powder inhaler. The inhaler may be a single or a multi dose inhaler, and may be a breath actuated dry powder inhaler.

One possibility is to mix the finely divided compound with a carrier substance, e.g. a mono-, di- or polysaccharide, a sugar alcohol, or an other polyol. Suitable carriers are sugars, e.g. lactose, glucose, raffinose, melezitose, lactitol, maltitol, trehalose, sucrose, mannitol; and starch. Alternatively the finely divided compound may be coated by another substance. The powder mixture may also be dispensed into hard gelatine capsules, each containing the desired dose of the active compound.

Another possibility is to process the finely divided powder into spheres which break up during the inhalation procedure. This spheronized powder may be filled into the drug reservoir of a multidose inhaler, e.g. that known as the Turbuhaler^{®} in which a dosing unit meters the desired dose which is then inhaled by the patient. With this system the active compound, with or without a carrier substance, is delivered to the patient.

For oral administration the active compound may be admixed with an adjuvant or a carrier, e.g. lactose, saccharose, sorbitol, mannitol; a starch, e.g. potato starch, corn starch or amylopectin; a cellulose derivative; a binder, e.g. gelatine or polyvinylpyrrolidone, and/or a lubricant, e.g. magnesium stearate, calcium stearate, polyethylene glycol, a wax, paraffin, and the like, and then compressed into tablets. If coated tablets are required, the cores, prepared as described above, may be coated with a concentrated sugar solution which may contain e.g. gum arabic, gelatine, talcum, titanium dioxide, and the like. Alternatively, the tablet may be coated with a suitable polymer dissolved in a readily volatile organic solvent.

For the preparation of soft gelatine capsules, the compound may be admixed with e.g. a vegetable oil or polyethylene glycol. Hard gelatine capsules may contain granules of the compound using either the above mentioned excipients for tablets. Also liquid or semisolid formulations of the drug may be filled into hard gelatine capsules.

Liquid preparations for oral application may be in the form of syrups or suspensions, for example solutions containing the compound, the balance being sugar and a mixture of ethanol, water, glycerol and propylene glycol. Optionally such liquid preparations may contain colouring agents, flavouring agents, saccharine and/or carboxymethylcellulose as a thickening agent or other excipients known to those skilled in art.

The compounds of the invention may also be administered in conjunction with other compounds used for the treatment of the above conditions.

The term 'medical therapy' as used herein is intended to include prophylactic, diagnostic and therapeutic regimens carried out in vivo or ex vivo on humans or other mammals.

The pharmaceutical compositions may be administered topically (e.g. to the lung and/or airways or to the skin) in the form of solutions, suspensions, heptafluoroalkane aerosols and dry powder formulations, or systemically, e.g. by oral administration in the form of tablets, capsules, syrups, powders or granules, or by parenteral administration in the form of solutions or suspensions, or by subcutaneous administration or by rectal administration in the form of suppositories or transdermally. Preferably the compound of the invention is administered orally.

The invention further relates to combination therapies wherein a compound of the invention or a pharmaceutically acceptable salts or solvate thereof, or a pharmaceutical composition or formulation comprising a compound of formula (1) is administered concurrently or sequentially with therapy and/or an agent for the treatment of any one of asthma, allergic rhinitis, cancer, COPD, rheumatoid arthritis, psoriasis, inflammatory bowel diseases, osteoarthritis or osteoporosis.

In particular, for the treatment of the inflammatory diseases rheumatoid arthritis, psoriasis, inflammatory bowel disease, COPD, asthma and allergic rhinitis the compounds of the invention may be combined with agents such as TNF-α inhibitors such as anti-TNF monoclonal antibodies (such as Remicade, CDP-870 and D₂E₇ and TNF receptor immunoglobulin molecules (such as Enbrel®), non-selective COX-1 / COX-2 inhibitors (such as piroxicam, diclofenac, propionic acids such as naproxen, flubiprofen, fenoprofen, ketoprofen and ibuprofen, fenamates such as mefenamic acid, indomethacin, sulindac, apazone, pyrazolones such as phenylbutazone, salicylates such as aspirin), COX-2 inhibitors (such as meloxicam, celecoxib, rofecoxib, valdecoxib and etoricoxib) low dose methotrexate, lefunomide, ciclesonide, hydroxychloroquine, d-penicillamine, auranofin or parenteral or oral gold.

The present invention still further relates to the combination of a compound of the invention together with a leukotriene biosynthesis inhibitor, 5-lipoxygenase (5-LO) inhibitor or 5-lipoxygenase activating protein (FLAP) antagonist such as zileuton, ABT-761, fenleuton, tepoxalin, Abbott-79175, Abbott-85761, N-(5-substituted)-thiophene-2-alkylsulfonamides, 2,6-di-tert-butylphenol hydrazones, methoxytetrahydropyrans such as Zeneca ZD-2138, the compound SB-210661, pyridinyl-substituted 2-cyanonaphthalene compounds such as L-739,010, 2-cyanoquinoline compounds such as L-746,530, indole and quinoline compounds such as MK-591, MK-886, and BAY x 1005.

The present invention still further relates to the combination of a compound of the invention together with a receptor antagonist for leukotrienes LTB₄, LTC₄, LTD₄, and LTE₄ selected from the group consisting of the phenothiazin-3-ones such as L-651,392, amidino compounds such as CGS-25019c, benzoxalamines such as ontazolast, benzenecarboximidamides such as BIIL 284/260, and compounds such as zafirlukast, ablukast, montelukast, pranlukast, verlukast (MK-679), RG-12525, Ro-245913, iralukast (CGP 45715A), and BAY x 7195.

The present invention still further relates to the combination of a compound of the invention together with a PDE4 inhibitor including inhibitors of the isoform PDE4D.

The present invention still further relates to the combination of a compound of the invention together with a antihistaminic H₂ receptor antagonists such as cetirizine, loratadine, desloratadine, fexofenadine, astemizole, azelastine, and chlorpheniramine.

The present invention still further relates to the combination of a compound of the invention together with a gastroprotective H₂. receptor antagonist.

The present invention still further relates to the combination of a compound of the invention together with an α₁.- and α₂.-adrenoceptor agonist vasoconstrictor sympathomimetic agent, such as propylhexedrine, phenylephrine, phenylpropanolamine, pseudoephedrine, naphazoline hydrochloride, oxymetazoline hydrochloride, tetrahydrozoline hydrochloride, xylometazoline hydrochloride, and ethylnorepinephrine hydrochloride.

The present invention still further relates to the combination of a compound of the invention together with anticholinergic agents such as ipratropium bromide, tiotropium bromide, oxitropium bromide, pirenzepine, and telenzepine.

The present invention still further relates to the combination of a compound of the invention together with a β₁- to β₄-adrenoceptor agonists such as metaproterenol, isoproterenol, isoprenaline, albuterol, salbutamol, formoterol, salmeterol, terbutaline, orciprenaline, bitolterol mesylate, and pirbuterol, or methylxanthanines including theophylline and aminophylline, sodium cromoglycate, or muscarinic receptor (M1, M2, and M3) antagonist.

The present invention still further relates to the combination of a compound of the invention together with an insulin-like growth factor type I (IGF-1) mimetic.

The present invention still further relates to the combination of a compound of the invention together with an inhaled glucocorticoid with reduced systemic side effects, such as prednisone, prednisolone, flunisolide, triamcinolone acetonide, beclomethasone dipropionate, budesonide, fluticasone propionate, and mometasone furoate.

The present invention still further relates to the combination of a compound of the invention together with an inhibitor of matrix metalloproteases (MMPs), i.e., the stromelysins, the collagenases, and the gelatinases, as well as aggrecanase, especially collagenase-1 (MMP-1), collagenase-2 (MMP-8), collagenase-3 (MMP-13), stromelysin-1 (MMP-3), stromelysin-2 (MMP-10), and stromelysin-3 (MMP-11) and MMP-12.

The present invention still further relates to the combination of a compound of the invention together with other modulators of chemokine receptor function such as CCR1, CCR2, CCR2A, CCR2B, CCR3, CCR4, CCR5, CCR6, CCR7, CCR8, CCR9, CCR10 and CCR11 (for the C-C family), CXCR1, CXCR3, CXCR4 and CXCR5 (for the C-X-C family) and CX₃CR1 for the C-X₃-C family.

The present invention still further relates to the combination of a compound of the invention together with antiviral agents such as Viracept, AZT, aciclovir and famciclovir, and antisepsis compounds such as Valant.

The present invention still further relates to the combination of a compound of the invention together with cardiovascular agents such as calcium channel blockers, lipid lowering agents such as statins, fibrates, beta-blockers, Ace inhibitors, Angiotensin-2 receptor antagonists and platelet aggregation inhibitors.

The present invention still further relates to the combination of a compound of the invention together with CNS agents such as antidepressants (such as sertraline), anti-Parkinsonian drugs (such as deprenyl, L-dopa, Requip, Mirapex, MAOB inhibitors such as selegine and rasagiline, comP inhibitors such as Tasmar, A-2 inhibitors, dopamine reuptake inhibitors, NMDA antagonists, Nicotine agonists, Dopamine agonists and inhibitors of neuronal nitric oxide synthase), and anti-Alzheimer's drugs such as donepezil, tacrine, COX-2 inhibitors, propentofylline or metryfonate.

The present invention still further relates to the combination of a compound of the invention together with (i) tryptase inhibitors, (ii) platelet activating factor (PAF) antagonists, (iii) interleukin converting enzyme (ICE) inhibitors, (iv) IMPDH inhibitors, (v) adhesion molecule inhibitors including VLA-4 antagonists, (vi) cathepsins, (vii) MAP kinase inhibitors, (viii) glucose-6 phosphate dehydrogenase inhibitors, (ix) kinin-B₁- and B₂-receptor antagonists, (x) anti-gout agents, e.g., colchicine, (xi) xanthine oxidase inhibitors, e.g., allopurinol, (xii) uricosuric agents, e.g., probenecid, sulfinpyrazone, and benzbromarone, (xiii) growth hormone secretagogues, (xiv) transforming growth factor (TGFβ), (xv) platelet-derived growth factor (PDGF), (xvi) fibroblast growth factor, e.g., basic fibroblast growth factor (bFGF), (xvii) granulocyte macrophage colony stimulating factor (GM-CSF), (xviii) capsaicin cream, (xix) Tachykinin NK₁ and NK₃ receptor antagonists selected from the group consisting of NKP-608C, SB-233412 (talnetant), and D-4418, (xx) elastase inhibitors selected from the group consisting of UT-77 and ZD-0892, (xxi) TNFα converting enzyme inhibitors (TACE), (xxii) induced nitric oxide synthase inhibitors (iNOS) or (xxiii) chemoattractant receptor-homologous molecule expressed on TH2 cells, (CRTH2 antagonists).

The compounds of the present invention may also be used in combination with osteoporosis agents such as roloxifene, droloxifene, lasofoxifene or fosomax and immunosuppressant agents such as FK-506, rapamycin, cyclosporine, azathioprine, and methotrexate.

The compounds of the invention may also be used in combination with existing therapeutic agents for the treatment of osteoarthritis. Suitable agents to be used in combination include standard non-steroidal anti-inflammatory agents (hereinafter NSAID's) such as piroxicam, diclofenac, propionic acids such as naproxen, flubiprofen, fenoprofen, ketoprofen and ibuprofen, fenamates such as mefenamic acid, indomethacin, sulindac, apazone, pyrazolones such as phenylbutazone, salicylates such as aspirin, COX-2 inhibitors such as celecoxib, valdecoxib, rofecoxib and etoricoxib, analgesics and intraarticular therapies such as corticosteroids and hyaluronic acids such as hyalgan and synvisc and P2X7 receptor antagonists.

The compounds of the invention can also be used in combination with existing therapeutic agents for the treatment of cancer. Suitable agents to be used in combination include:
(i) antiproliferative/antineoplastic drugs and combinations thereof, as used in medical oncology, such as alkylating agents (for example cis-platin, carboplatin, cyclophosphamide, nitrogen mustard, melphalan, chlorambucil, busulphan and nitrosoureas), antimetabolites (for example antifolates such as fluoropyrimidines like 5-fluorouracil and tegafur, raltitrexed, methotrexate, cytosine arabinoside, hydroxyurea, gemcitabine and paclitaxel (Taxol®), antitumour antibiotics (for example anthracyclines like adriamycin, bleomycin, doxorubicin, daunomycin, epirubicin, idarubicin, mitomycin-C, dactinomycin and mithramycin), antimitotic agents (for example vinca alkaloids like vincristine, vinblastine, vindesine and vinorelbine and taxoids like taxol and taxotere), and topoisomerase inhibitors (for example epipodophyllotoxins like etoposide and teniposide, amsacrine, topotecan and camptothecin),
(ii) cytostatic agents such as antioestrogens (for example tamoxifen, toremifene, raloxifene, droloxifene and iodoxyfene), oestrogen receptor down regulators (for example fulvestrant), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5α-reductase such as finasteride,
(iii) Agents which inhibit cancer cell invasion (for example metalloproteinase inhibitors like marimastat and inhibitors of urokinase plasminogen activator receptor function),
(iv) inhibitors of growth factor function, for example such inhibitors include growth factor antibodies, growth factor receptor antibodies (for example the anti-erbb2 antibody trastuzumab [Herceptin™] and the anti-erbb1 antibody cetuximab [C225]), farnesyl transferase inhibitors, tyrosine kinase inhibitors and serine/threonine kinase inhibitors, for example inhibitors of the epidermal growth factor family (for example EGFR family tyrosine kinase inhibitors such as N-(3-chloro-4-fluorophenyl)-7-methoxy-6-(3-morpholinopropoxy)quinazolin-4-amine (gefitinib, AZD1839), N-(3-ethynylphenyl)-6,7-bis(2-methoxyethoxy)quinazolin-4-amine (erlotinib, OSI-774) and 6-acrylamido-N-(3-chloro-4-fluorophenyl)-7-(3-morpholinopropoxy)quinazolin-4-amine (CI 1033)), for example inhibitors of the platelet-derived growth factor family and for example inhibitors of the hepatocyte growth factor family,
(v) antiangiogenic agents such as those which inhibit the effects of vascular endothelial growth factor, (for example the anti-vascular endothelial cell growth factor antibody bevacizumab [Avastin™], compounds such as those disclosed in International Patent Applications WO 97/22596, WO 97/30035, WO 97/32856 and WO 98/13354) and compounds that work by other mechanisms (for example linomide, inhibitors of integrin αvβ3 function and angiostatin),
(vi) vascular damaging agents such as Combretastatin A4 and compounds disclosed in International Patent Applications WO 99/02166, WO00/40529, WO 00/41669, WO01/92224, WO02/04434 and WO02/08213,
(vii) antisense therapies, for example those which are directed to the targets listed above, such as ISIS 2503, an anti-ras antisense,
(viii) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy, and
(ix) immunotherapy approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

The following Examples illustrate the invention.

*General methods* All reactions were performed in dried glassware in an argon atmosphere at room temperature, unless otherwise noted. All solvents and reagents and solvents were used as received. Merck Silica gel 60 (0.040-0.063 mm) was used for preparative silica gel chromatography. A Kromasil KR-100-5-C18 column (250 x 20 mm, Akzo Nobel) and mixtures of acetonitrile/water at a flow rate of 10 ml/min was used for preparative HPLC. Reactions were monitored at 254 nm by analytical HPLC, using a Kromasil C-18 column (150 x 4.6 mm) and a gradient (containing 0.1% trifluoroacetic acid) of 5 to 100% of acetonitrile in water at a flow rate of 1 ml/min. Evaporations of solvents were performed under reduced pressure using a rotary evaporator at a maximum temperature of 40°C. Products were dried under reduced pressure at 40°C.
¹H-NMR spectra were recorded on a Varian Inova-400 or Unity-500+ instrument. The central solvent peak of chloroform-*d* (□_{H} 7.27 ppm), dimethylsulfoxide-*d*₆ (□_{H} 2.50 ppm) or methanol-*d*₄ (□_{H} 3.35 ppm) were used as internal references. Low resolution mass spectra obtained on a Hewlett Packard 1100 LC-MS system equipped with a APCI ionisation chamber.

Merck Silica gel 60 (0.040-0.063 mm) was used for preparative silica gel chromatography. A Kromasil KR-100-5-C18 column (250 x 20 mm, Akzo Nobel) and mixtures of acetonitrile/water at a flow rate of 10 ml/min was used for preparative HPLC. Reactions were monitored at 254 nm by analytical HPLC, using a Kromasil C-18 column (150 x 4.6 mm) and a gradient (containing 0.1 % trifluoroacetic acid) of 5 to 100% of acetonitrile in water at a flow rate of 1 ml/min. Evaporations of solvents were performed under reduced pressure using a rotary evaporator at a maximum temperature of 40 °C. Products were dried under reduced pressure at 40 °C.

### Thiazolopyridines

### Example 1

### 7-[(2-Ethylphenyl)amino]-2-methoxy[1,3]thiazolo[5,4-b]pyridine-6-carboxamide

### 1a) 7-Hydroxy-2-(methylthio)[1,3]thiazolo[5,4-b]pyridine-6-carboxylic acid

The title compound was synthesized according to procedure described by D.C. Leysen; A. Haemers; W. Bollaert, J. Heterocyclic Chem. 1984, 21, 401.

### 1b) 7-Chloro-2-(methylthio)[1,3]thiazolo[5,4-b]pyridine-6-carboxamide

To a suspension of finely grinded **1a** (3.70 g, 15.3 mmol) in thionyl chloride (100 mL) was added a drop of DMF. The suspension was stirred at 50 C for 4h. The thionyl chloride was evaporated. Toluene was added. After a short time of stirring the toluene was evaporated. Acetone (125 mL) was added to the crude residue to form a suspension which was stirred and cooled to 0 C. Cold Ammonium hydroxide (25%, 20 mL, 306 mmol) was added and the reaction was stirred for 1h and put in a refrigerator over night. The precipitate was filtered off and washed twice with water and dried under vacuum at 70 °C over night.
Yield: 2.86g (72%).
¹H NMR (400 MHz, DMSO-d₆): δ 8.52 (s, 1H), 8.14 (s, 1H), 7.91 (s, 1H), 2.85 (s, 3H). APCI-MS m/z: 259.9 [MH+].

### 1c) 7-[(2-Ethylphenyl)amino]-2-(methylthio)[1,3]thiazolo[5,4-b]pyridine-6-carboxamide

To a solution of **1b** (2.00 g, 7.7 mmol) and 2-ethylaniline (2.9 mL, 23.1 mmol) in DMSO (30 mL) was added BF₃.OEt₂ (0.95mL, 7.7 mmol). The solution was stirred over night at 110 C. After cooling the reaction was poured into ice water and EtOAc. sodium carbonate (5 %) was added to obtain an alkaline solution. The organic phase was separated and the water phase extracted twice with EtOAc. The combined organic phases were washed with three portions of water and brine. Dried over sodium sulphate and purified by flash chromatography (toluene:EtOAc 1:1).
Yield 1.98g, 5.75 mmol (75%).
¹H NMR (400 MHz, DMSO-d₆) δ 10.99 (s, 1H), 8.67 (s, 1H), 8.31 (s, 1H), 7.70 (s, 1H), 7.27 - 7.25 (m, 1H), 7.16 - 7.13 (m, 2H), 7.09 - 7.07 (m, 1H), 2.58 (q, *J =* 7.5 Hz, 2H), 2.16 (s, 3H), 1.13 (t, *J*= 7.5 Hz, 3H).
APCI-MS m/z: 345.2 [MH+].

### 1d) 7-[(2-Ethylphenyl)amino]-2-(methylsulfinyl)[1,3]thiazolo[5,4-b]pyridine-6-carboxamide

A suspension of **1c** (1.00 g, 2.91 mmol) stirred in dichloromethane (15 mL) was cooled to -15 °C. 3-Chloroperbenzoic acid (680 mg, 3.05 mmol) was added portion wise. The reaction was stirred 1h, allowed to reach room temperature and quenched with sodium thiosulfate (5 %). The organic phase was separated, washed with sodium bicarbonate and brine and dried over sodium sulphate. The product was purified by flash chromatography (EtOAc as eluent).
Yield: 910 mg, 2.52 mmol (87%).
¹H NMR (400 MHz, DMSO-d₆) δ 11.28 (s, 1H), 8.84 (s, 1H), 8.40 (s, 1H), 7.78 (s, 1H), 7.27 - 7.26 (m, 1H), 7.20 - 7.15 (m, 1H), 7.14 - 7.10 (m, 2H), 2.70 (s, 3H), 2.57 (q, *J*= 7.4 Hz, 2H), 1.11 (t, *J* = 7.6 Hz, 3H).
APCI-MS M/Z: 361.1 [MH+].

### 1e) 7-[(2-Ethylphenyl)amino]-2-(methylsulfonyl)[1,3]thiazolo[5,4-b]pyridine-6-carboxamide

Compound **1d** (600 mg, 1.74 mmol) was dissolved in HOAc (16 mL). The solution was cooled in an ice bath. When the solution started to freeze a slow addition of potassium permanganate (560 mg, 3.52 mmol) dissolved in water (12 mL) was started. After 20 min the addition was completed. The stirring was continued for an additional 30 min. A saturated solution of sodium bisulphite (2.0 mL) was added. A precipitate was formed. The suspension was centrifuged and the supernatant was collected and extracted with chloroform. The organic layer was separated, washed twice with sodium bicarbonate (5%) and brine, and dried over sodium sulphate. The product was purified by flash-chromatography (toluene: EtOAc 4:6).
Yield: 101 mg (15%).
APCI-MS m/z: 377.2 [MH+].

### 1f) 7-[(2-Ethylphenyl)amino]-2-methoxy[1,3]thiazolo[5,4-b]pyridine-6-carboxamide 1e

(20 mg, 0.052 mmol) was placed in a dry reaction flask. Sodium methoxide (0.35M in methanol, 0.16 mL, 560 µmol) was added. The reaction was stirred at room temperature for 2h and then quenched by adding water. The mixture was extracted with chloroform. The organic layer was separated and washed twice with water and dried over sodium sulphate. The product was purified by flash chromatography (toluene:EtOAc 1:1).
Yield: 10 mg (59%).
¹H NMR (400 MHz, DMSO-d₆) δ 10.79 (s, 1H), 8.62 (s, 1H), 8.28 (s, 1H), 7.67 (s, 1H), 7.23 (dd, *J* = 7.2, 1.9 Hz, 1H), 7.12 (qd, *J* = 7.3, 5.4 Hz, 2H), 7.02 (dd, *J* = 7.3, 1.7 Hz, 1H), 3.60 (s, 3H), 2.59 (q, *J*= 7.8 Hz, 2H), 1.15 (t, *J*= 7.6 Hz, 3H).
APCI-MS m/z: 329.2 [MH+].

### Example 2

### 7-[(2-methylphenyl)amino]-2-(methylthio)[1,3]thiazolo [5,4-b] pyridine-6-carboxamide trifluoroacetate

The title compound was prepared in an analogues way to **1c** (100 mg, 0.38 mmol), 2-methylaniline (0.123 mL, 1.15 mmol) and boron trifluoride ethyl etherate (0.047 mL, 0.38 mmol) in DMSO (2 mL). The product was purified by prep-HPLC.
Yield: 17 mg, 0.038 mmol (10%).
APCI-MS m/z: 331.2 [MH+]

### Example 3

### 7-([3-(hydroxymethyl)-2-methylphenyl]amino)-2-(methylthio)[1,3]thiazolo[5,4-b]pyridine-6-carboxamide trifluoroacetate

The title compound was prepared in an analogues way to **1c** (100 mg, 0.38 mmol), 3-(hydroxymethyl)-2-methylaniline (158 mg, 1.15 mmol) and boron trifluoride ethyl etherate (0.047 mL, 0.38 mmol) in dmso (2 mL). The product was purified by prep-HPLC.
Yield: 53 mg, 0.11 mmol (29%).
APCI-MS m/z: 361.2 [MH+]

Example **4-7** were prepared in an analogues way to **1f.**

### Example 4

### 2-[2-(Dimethylamino)ethoxy]-7-[(2-ethylphenyl)amino] [1,3]thiazolo[5,4-b]pyridine-6-carboxamide

APCI-MS m/z: 386.2 [MH+].

### Example 5

### _2-[3-(Dimethylamino)propoxy]-7-[(2-ethylphenyl)amino][1,3]thiazolo[5,4-b]pyridine-6-carboxamide

APCI-MS m/z: 400.2 [MH+].

### Example 6

### 7-[(2-Ethylphenyl)amino]-2-(2-morpholin-4-ylethoxy)[1,3] thiazolo [5,4-b] pyridine-6-carboxamide

APCI-MS m/z: 428.2 [MH+].

### Example 7

### 7-[(2-Ethylphenyl)amino]-2-[(3-morpholin-4-ylpropyl)amino] [1,3]thiazolo[5,4-b]pyridine-6-carboxamide

APCI-MS m/z: 441.2 [MH+].

Example **8-10** were prepared in an analogues way to example **1f** using **1d** as reactant and at slightly elevated temperature (50°C).

### Example 8

### 2-[4-(Aminocarbonyl)phenoxy]-7-[(2-ethylphenyl)amino][1,3]thiazolo [5,4b]pyridine-6-carboxamide

APCI-MS m/z: 434.1 [MH+].

### Example 9

### 2-(3-aminophenoxy)-7-[(2-ethylphenyl)amino] [1,3]thiazolo[5,4-b] pyridine-6-carboxamide bis(trifluoroacetate)

APCI-MS m/z: 406.1 [MH+].

### Example 10

### 7-[(2-ethylphenyl)amino]-2-(4-methoxyphenoxy)[1,3]thiazolo[5,4-b] pyridine-6-carboxamide trifluoroacetate

APCI-MS m/z: 421.1 [MH+].

### Imidazopyridines

### Example 1

### 7-{[2-ethyl-3-(hydroxymethyl)phenyl]amino}-2-phenyl-3H-imidazo[4,5-b] pyridine-6-carboxamide trifluoroacetate (salt)

### 1a) Methyl 4,6-dichloro-5-nitronicotinate

The title compound was prepered according to the protocol of J. P. Sanchez and R. D. Gogliottias, J. Heterocyclic Chem. 1993, 30, 855-859.

### 1b) Methyl 6-chloro-4-{[2-ethyl-3-(hydroxymethyl)phenyl]amino}-5-nitronicotinate A

solution of Methyl 4,6-dichloro-5-nitronicotinate (603 mg, 2.40 mmol), 2-Ethyl-3-hydroxymethyl-aniline (363 mg, 2,40 mmol, 1.0 equiv.) and ethyl(di*iso*propyl)amine (373 mg, 490 □L, 288 mmol, 1.2 equiv.) was stirred at 50°C over night. Addition of water, extraction with ethylacetate followed by washing with brine and drying over sodium sulfate resulted in a crude product. After chromatography on silica gel [methyl(*tert-*butyl)ether/heptane = 4:1] 466 mg of the title compound were obtained as a yellow solid. ¹H-NMR (400MHz, CDCl₃): δ 10.37 (s, 1H), 8.85 (s, 1H), 7.39 (d, *J* 7.1Hz, 1H), 7.14 (t, *J* 7.9Hz, 1H), 6.97 (d, *J* 7.7Hz, 1H), 4.76 (s, 2H), 4.00 (s, 3H), 2.75 (q, *J* 7.5Hz, 2H), 1.22 (t, *J*= 7.7Hz, 3H).

### 1c) 6-Amino-4-{[2-ethyl-3-(hydroxymethyl)phenyl]amino}-5-nitronicotinamide

The reaction was carried out in a sealed steal tube equiped with a glass inlet. Compound **1b** (436 mg, 1.19 mmol) was placed in the reaction vessel and liquid ammonia was condensed in at -78°C. Then the mixture was allowed to warm up to room temperature. With a delay of 12 h the reaction mixture was warmed up to 80°C for additional 8 h. Then the reaction vessel was cooled down to -78°C, opened and ammonia was allowed to evaporate. Filtration of the crude on silica gel (EtOAc/Aceton = 9:1) afforded 239 mg (61%) of the pure title compound as a yellow solid.
¹H-NMR (400MHz, DMSO-d₆): δ 11.84 (s, 1H), 8.49 (s, 1H), 8.10 (s, 1H), 7.49 (s, 1H), 7.39 (s, 2H), 7.13 (d, *J* 7.0Hz, 1H), 6.98 (t, *J* 7.8Hz, 1H), 6.69 (d, *J* 7.3Hz, 1H), 5.14 (t, *J* 5.5Hz, 1H), 4.54 (d, *J* 5.5Hz, 2H), 2.64 (q, *J* 7.5Hz, 2H), 1.14 (t, *J* 7.3Hz, 3H).

### 1d) 5,6-Diamino-4-{[2-ethyl-3-(hydroxymethyl)phenyl]amino}nicotinamide

Compound **1c** (2.22g, 6.71 mmol) was dissolved in methanol (40 ml) and palladium (10% on charcoal, 450 mg) was added. The mixture was stirred under normal pressure under a hydrogen atmospereat at room temperature over night. Filtration over celite and evaporation of the solvent resulted in the title compound as a colourless solid in quantitative yields.
¹H-NMR (400MHz, DMSO-d₆): δ 9.17 (s, 1H), 7.88 (s, 1H), 7.80 (s, 1H), 7.14 (s, 1H), 6.98 (t, *J* 7.7Hz, 1H), 6.92 (d, *J* 6.7Hz, 1H), 6.16 (d, *J* 7.4Hz, 1H), 5.90 (s, 2H), 5.03 (t, *J* 5.1Hz, 1H), 4.53 (d, *J* 4.9Hz, 2H), 3.72 (s, 2H), 2.72 (q, *J*= 7.5Hz, 2H), 1.21 (t, *J* 7.4Hz, 3H).
APCI-MS: m/z 302.2 [MH⁺].

### 1e) 7-{[2-ethyl-3-(hydroxymethyl)phenyl]amino}-2-phenyl-3H-imidazo[4,5-b]pyridine-6-carboxamide trifluoroacetate (salt) A

solution of compound **1d** (18 mg, 59.0 µmol), benzaldehyde (7mg, 6.6 µL, 66.0 µmol, 1.1 equiv.) and a catalytic amount of *p*-toluensulfonic acid monohydrate in DMF (400µL) was strirred at 120°C for 1h. After cooling down the mixture was diluted with acetonitrile (2.5 mL) and water (2mL) and subjected to a reversed phase HPLC. After freezedrying 8 mg (27 %) of the title compound as its corresponding trifluoroacetate were obtained as a white powder. The NMR was recorded from the neutralized product.
¹H-NMR (400MHz, DMSO-d₆): δ 13.30 (s,1H), 11.13 (s, 1H), 8.59 (s, 1H), 8.10 (s. 1H), 7.40 (m, 3H), 7.67 (m, 1H), 7.40 (m, 3H), 7.18 (m, 2H), 7.09 (t, *J* 7.4Hz, 1H), 5.10 (t, *J* 5.0Hz, 1H), 4.58 (d, *J* 4.7Hz), 2.72 (q, *J*7.3Hz, 2H), 1.15 (t, *J*7.2Hz, 3H).
APCI-MS: m/z 388.1 [MH⁺].

The title compounds of examples **2-26** were prepared according to the procedure described for example **1.** In some cases FeCl₃ instead of *p*-toluensulfonic acid was used to catalyze the reaction of step **1e.**
7-[(2-ethylphenyl)amino]-2-(4-hydroxyphenyl)-3H-imidazo[4,5-b]pyridine-6-carboxamide 2-[4-(dimethylamino)phenyl]-7-[(2-ethylphenyl)amino]-3H-imidazo[4,5-b]pyridine-6-carboxamide bis(trifluoroacetate)
7-[(2-ethylphenyl)amino]-2-(3-hydroxyphenyl)-3H-imidazo[4,5-b]pyridine-6-carboxamide trifluoroacetate (salt)
7-[(2-ethylphenyl)amino]-2-(4-methoxyphenyl)-3H-imidazo[4,5-b]pyridine-6-carboxamide trifluoroacetate
2-(3,4-dihydroxyphenyl)-7-[(2-ethylphenyl)amino]-3H-imidazo[4,5-b]pyridine-6-carboxamide trifluoroacetate (salt)
2-(3-cyanophenyl)-7-[(2-ethylphenyl)amino]-3H-imidazo[4,5-b]pyridine-6-carboxamide trifluoroacetate
2-(4-cyanophenyl)-7-[(2-ethylphenyl)amino]-3H-imidazo[4,5-b]pyridine-6-carboxamide trifluoroacetate
7-[(2-ethylphenyl)amino]-2-(4-hydroxy-3-methoxyphenyl)-3H-imidazo[4,5-b]pyridine-6-carboxamide trifluoroacetate (salt)
2-(3,4-difluorophenyl)-7-[(2-ethylphenyl)amino]-3H-imidazo[4,5-b]pyridine-6-carboxamide trifluoroacetate
2-(3-chloro-4-hydroxyphenyl)-7-[(2-ethylphenyl)amino]-3H-imidazo[4,5-b]pyridine-6-carboxamide trifluoroacetate (salt)
7-[(2-ethylphenyl)amino]-2-(1H-imidazol-4-yl)-3H-imidazo[4,5-b]pyridine-6-carboxamide bis(trifluoroacetate)
7-[(2-ethylphenyl)amino]-2-[4-(methylsulfonyl)phenyl]-3H-imidazo[4,5-b]pyridine-6-carboxamide trifluoroacetate
2-[4-(acetylamino)phenyl]-7-[(2-ethylphenyl)amino]-3H-imidazo[4,5-b]pyridine-6-carboxamide trifluoroacetate
7-[(2-ethylphenyl)amino]-2-[4-(2-hydroxyethoxy)phenyl]-3H-imidazo[4,5-b]pyridine-6-carboxamide trifluoroacetate (salt)
7-[(2-ethylphenyl)amino]-2- {4-[(2-hydroxyethyl)(methyl)amino]phenyl}-3H-imidazo[4,5-b]pyridine-6-carboxamide bis(trifluoroacetate) (salt)
7-[(2-ethylphenyl)amino]-2-(4-morpholin-4-ylphenyl)-3H-imidazo[4,5-b]pyridine-6-carboxamide bis(trifluoroacetate)
7-[(2-ethylphenyl)amino]-2-phenyl-3H-imidazo[4,5-b]pyridine-6-carboxamide trifluoroacetate
7- {[2-ethyl-3-(hydroxymethyl)phenyl]amino}-2-(4-hydroxyphenyl)-3H-imidazo[4,5-b]pyridine-6-carboxamide trifluoroacetate (salt)
7- {[2-ethyl-3-(hydroxymethyl)phenyl]amino}-2-(4-morpholin-4-ylphenyl)-3H-imidazo[4,5-b]pyridine-6-carboxamide
7-[(2-ethylphenyl)amino]-2-pyridin-4-yl-3H-imidazo[4,5-b]pyridine-6-carboxamide trifluoroacetate
7-[(2-ethylphenyl)amino]-2-pyridin-3-yl-3H-imidazo[4,5-b]pyridine-6-carboxamide trifluoroacetate
7-[(2-ethylphenyl)amino]-2-pyridin-2-yl-3H-imidazo[4,5-b]pyridine-6-carboxamide trifluoroacetate
2-[4-(2- {[tert-butyl(diphenyl)silyl]oxy}ethoxy)phenyl]-7-{[2-ethyl-3-(hydroxymethyl)phenyl]amino}-3H-imidazo[4,5-b]pyridine-6-carboxamide
7- {[2-ethyl-3-(hydroxymethyl)phenyl]amino}-2-pyridin-4-yl-3H-imidazo[4,5-b]pyridine-6-carboxamide
7- {[2-ethyl-3-(hydroxymethyl)phenyl]amino}-2-(4-methoxyphenyl)-3H-imidazo[4,5-b]pyridine-6-carboxamide

### Example 27

### 7-[(2-ethyl-3-{[(2-hydroxypropyl)amino]methyl}phenyl)amino]-2-(4-methoxyphenyl)-3H-imidazo[4,5-b]pyridine-6-carboxamide

Treatment of a suspension of example **26** in dichloromethane with thionyl chloride at room temperature generated the correponding chloride. The volatiles were removed in vaccuo, and the residue was dissolved in NMP. The chloride (0.1M) was then treated with 1.5 eq of 1-amino-propan-2-ol (0.2M in NMP) and 3eq of DIPEA(0.2M in NMP) and heated at 120 °C for 12h generating the title compound in good yield.

The title compounds of examples **28-93** were prepared in analogous manner to example **26**:

### Examples 28 - 93

7-[(2-ethyl-3-{[(2-hydroxypropyl)amino]methyl}phenyl)amino]-2-(4-methoxyphenyl)-3H-imidazo[4,5-b]pyridine-6-carboxamide
7-[(2-ethyl-3- {[(2-hydroxypropyl)amino]methyl}phenyl)amino]-2-[4-(2-hydroxyethoxy)phenyl]-3H-imidazo[4,5-b]pyridine-6-carboxamide
7-[(2-ethyl-3- {[(2-hydroxy-1-methylethyl)amino]methyl}phenyl)amino]-2-[4-(2-hydroxyethoxy)phenyl]-3H-imidazo[4,5-b]pyridine-6-carboxamide
7-{[2-ethyl-3-(1H-imidazol-1-ylmethyl)phenyl]amino}-2-[4-(2-hydroxyethoxy)phenyl]-3H-imidazo[4,5-b]pyridine-6-carboxamide
7-[(3-{[(2,3-dihydroxypropyl)amino]methyl}-2-ethylphenyl)amino]-2-[4-(2-hydroxyethoxy)phenyl]-3H-imidazo[4,5-b]pyridine-6-carboxamide
7- {[2-ethyl-3-(morpholin-4-ylmethyl)phenyl]amino}-2-[4-(2-hydroxyethoxy)phenyl]-3H-imidazo[4,5-b]pyridine-6-carboxamide

7-{[3-({[(1S)-2-cyclohexyl-1-(hydroxymethyl)ethyl]amino}methyl)-2-ethylphenyl]amino}-2-[4-(2-hydroxyethoxy)phenyl]-3H-imidazo[4,5-b]pyridine-6-carboxamide
7-{[3-({[(1S)-1-benzyl-2-hydroxyethyl]amino}methyl)-2-ethylphenyl]amino}-2-[4-(2-hydroxyethoxy)phenyl]-3H-imidazo[4,5-b]pyridine-6-carboxamide
7-[(2-ethyl-3-{[4-(2-hydroxyethyl)piperazin-1-yl]methyl}phenyl)amino]-2-[4-(2-hydroxyethoxy)phenyl]-3H-imidazo[4,5-b]pyridine-6-carboxamide

7-({2-ethyl-3-[(3-hydroxypyrrolidin-1-yl)methyl]phenyl}amino)-2-[4-(2-hydroxyethoxy)phenyl]-3H-imidazo[4,5-b]pyridine-6-carboxamide
7- {[2-ethyl-3-({[(1S)-2-hydroxy-1-phenylethyl]amino}methyl)phenyl]amino}-2-[4-(2-hydroxyethoxy)phenyl]-3H-imidazo[4,5-b]pyridine-6-carboxamide
7-[(2-ethyl-3- {[(2R)-2-(hydroxymethyl)pyrrolidin-1-yl]methyl}phenyl)amino]-2-[4-(2-hydroxyethoxy)phenyl]-3H-imidazo[4,5-b]pyridine-6-carboxamide
7- {[2-ethyl-3-({[(1S,2S)-2-hydroxy-1-(hydroxymethyl)-2-phenylethyl]amino}methyl)phenyl]amino}-2-[4-(2-hydroxyethoxy)phenyl]-3H-imidazo[4,5-b]pyridine-6-carboxamide
7-[(2-ethyl-3-{[(2-hydroxycyclohexyl)amino]methyl}phenyl)amino]-2-[4-(2-hydroxyethoxy)phenyl]-3H-imidazo[4,5-b]pyridine-6-carboxamide
7- {[2-ethyl-3-({[(1R,2S)-2-hydroxy-2-(4-hydroxyphenyl)-1-methylethyl]amino}methyl)phenyl]amino}-2-[4-(2-hydroxyethoxy)phenyl]-3H-imidazo[4,5-b]pyridine-6-carboxamide
7- {[2-ethyl-3-({[(1R,2R)-2-hydroxy-1-(hydroxymethyl)-2-phenylethyl]amino}methyl)phenyl]amino}-2-[4-(2-hydroxyethoxy)phenyl]-3H-imidazo[4,5-b]pyridine-6-carboxamide

7-({2-ethyl-3-[(3-hydroxypiperidin-1-yl)methyl]phenyl}amino)-2-[4-(2-hydroxyethoxy)phenyl]-3H-imidazo[4,5-b]pyridine-6-carboxamide
7-{[2-ethyl-3-({[1-(hydroxymethyl)-2-methylpropyl]amino}methyl)phenyl]amino}-2-[4-(2-hydroxyethoxy)phenyl]-3H-imidazo[4,5-b]pyridine-6-carboxamide
7-{[2-ethyl-3-({[4-(methylsulfonyl)benzyl]amino}methyl)phenyl]amino}-2-[4-(2-hydroxyethoxy)phenyl]-3H-imidazo[4,5-b]pyridine-6-carboxamide
7-{[3-({[2-(3,4-dihydroxyphenyl)-2-hydroxyethyl]amino}methyl)-2-ethylphenyl]amino}-2-(4-methoxyphenyl)-3H-imidazo[4,5-b]pyridine-6-carboxamide
7-[(2-ethyl-3-{[(2-hydroxypropyl)amino]methyl}phenyl)amino]-2-(4-methoxyphenyl)-3H-imidazo[4,5-b]pyridine-6-carboxamide

7-[(2-ethyl-3-{[(2-hydroxy-1-methylethyl)amino]methyl}phenyl)amino]-2-(4-methoxyphenyl)-3H-imidazo[4,5-b]pyridine-6-carboxamide
7-{[2-ethyl-3-(1H-imidazol-1-ylmethyl)phenyl]amino}-2-(4-methoxyphenyl)-3H-imidazo[4,5-b]pyridine-6-carboxamide trifluoroacetate
7-[(3-{[(2,3-dihydroxypropyl)amino]methyl}-2-ethylphenyl)amino]-2-(4-methoxyphenyl)-3H-imidazo[4,5-b]pyridine-6-carboxamide
7-{[2-ethyl-3-(morpholin-4-ylmethyl)phenyl]amino}-2-(4-methoxyphenyl)-3H-imidazo[4,5-b]pyridine-6-carboxamide trifluoroacetate
7-{[3-({[(1S)-2-cyclohexyl-1-(hydroxymethyl)ethyl]amino}methyl)-2-ethylphenyl]amino}-2-(4-methoxyphenyl)-3H-imidazo[4,5-b]pyridine-6-carboxamide
7-{[3-({[(1S)-1-benzyl-2-hydroxyethyl]amino}methyl)-2-ethylphenyl]amino}-2-(4-methoxyphenyl)-3H-imidazo[4,5-b]pyridine-6-carboxamide
7-[(2-ethyl-3-{[4-(2-hydroxyethyl)piperazin-1-yl]methyl}phenyl)amino]-2-(4-methoxyphenyl)-3H-imidazo[4,5-b]pyridine-6-carboxamide
7-({2-ethyl-3-[(3-hydroxypyrrolidin-1-yl)methyl]phenyl}amino)-2-(4-methoxyphenyl)-3H-imidazo[4,5-b]pyridine-6-carboxamide
7- {[2-ethyl-3-({[(1S)-2-hydroxy-1-phenylethyl]amino}methyl)phenyl]amino}-2-(4-methoxyphenyl)-3H-imidazo[4,5-b]pyridine-6-carboxamide
7-[(2-ethyl-3- {[(2R)-2-(hydroxymethyl)pyrrolidin-1-yl]methyl}phenyl)amino]-2-(4-methoxyphenyl)-3H-imidazo[4,5-b]pyridine-6-carboxamide
7- {[2-ethyl-3-({[(1S,2S)-2-hydroxy-1-(hydroxymethyl)-2-phenylethyl]amino}methyl)phenyl]amino}-2-(4-methoxyphenyl)-3H-imidazo[4,5-b]pyridine-6-carboxamide
7-[(2-ethyl-3-{[(2-hydroxycyclohexyl)amino]methyl}phenyl)amino]-2-(4-methoxyphenyl)-3H-imidazo[4,5-b]pyridine-6-carboxamide
7- {[2-ethyl-3-({[(1R,2S)-2-hydroxy-2-(4-hydroxyphenyl)-1-methylethyl]amino}methyl)phenyl]amino}-2-(4-methoxyphenyl)-3H-imidazo[4,5-b]pyridine-6-carboxamide
7- {[2-ethyl-3-({[(1R,2R)-2-hydroxy-1-(hydroxymethyl)-2-phenylethyl]amino}methyl)phenyl]amino}-2-(4-methoxyphenyl)-3H-imidazo[4,5-b]pyridine-6-carboxamide
7-({2-ethyl-3-[(3-hydroxypiperidin-1-yl)methyl]phenyl}amino)-2-(4-methoxyphenyl)-3H-imidazo[4,5-b]pyridine-6-carboxamide
7- {[2-ethyl-3-({[1-(hydroxymethyl)-2-methylpropyl]amino}methyl)phenyl]amino}-2-(4-methoxyphenyl)-3H-imidazo[4,5-b]pyridine-6-carboxamide

7- {[2-ethyl-3-({[4-(methylsulfonyl)benzyl]amino}methyl)phenyl]amino}-2-(4-methoxyphenyl)-3H-imidazo[4,5-b]pyridine-6-carboxamide trifluoroacetate
7-[(2-ethyl-3- {[(2-hydroxypropyl)amino]methyl}phenyl)amino]-2-(4-morpholin-4-ylphenyl)-3H-imidazo[4,5-b]pyridine-6-carboxamide
7-[(2-ethyl-3- {[(2-hydroxy-1-methylethyl)amino]methyl}phenyl)amino]-2-(4-morpholin-4-ylphenyl)-3H-imidazo[4,5-b]pyridine-6-carboxamide
7- {[2-ethyl-3-(1H-imidazol-1-ylmethyl)phenyl]amino}-2-(4-morpholin-4-ylphenyl)-3H-imidazo[4,5-b]pyridine-6-carboxamide trifluoroacetate
7-[(3- {[(2,3-dihydroxypropyl)amino]methyl}-2-ethylphenyl)amino]-2-(4-morpholin-4-ylphenyl)-3H-imidazo[4,5-b]pyridine-6-carboxamide
7-{[2-ethyl-3-(morpholin-4-ylmethyl)phenyl]amino}-2-(4-morpholin-4-ylphenyl)-3H-imidazo[4,5-b]pyridine-6-carboxamide trifluoroacetate
7-{[3-({[(1S)-1-benzyl-2-hydroxyethyl]amino}methyl)-2-ethylphenyl]amino}-2-(4-morpholin-4-ylphenyl)-3H-imidazo[4,5-b]pyridine-6-carboxamide
7-[(2-ethyl-3-{[4-(2-hydroxyethyl)piperazin-1-yl]methyl}phenyl)amino]-2-(4-morpholin-4-ylphenyl)-3H-imidazo[4,5-b]pyridine-6-carboxamide
7-({2-ethyl-3-[(3-hydroxypyrrolidin-1-yl)methyl]phenyl}amino)-2-(4-morpholin-4-ylphenyl)-3H-imidazo[4,5-b]pyridine-6-carboxamide
7- {[2-ethyl-3-({[(1S)-2-hydroxy-1-phenylethyl]amino}methyl)phenyl]amino}-2-(4-morpholin-4-ylphenyl)-3H-imidazo[4,5-b]pyridine-6-carboxamide
7-[(2-ethyl-3- {[(2R)-2-(hydroxymethyl)pyrrolidin-1-yl]methyl}phenyl)amino]-2-(4-morpholin-4-ylphenyl)-3H-imidazo[4,5-b]pyridine-6-carboxamide
7- {[2-ethyl-3-({[(1S,2S)-2-hydroxy-1-(hydroxymethyl)-2-phenylethyl] amino}methyl)phenyl]amino}-2-(4-morpholin-4-ylphenyl)-3H-imidazo[4,5-b]pyridine-6-carboxamide
7-[(2-ethyl-3- {[(2-hydroxycyclohexyl)amino]methyl}phenyl)amino]-2-(4-morpholin-4-ylphenyl)-3H-imidazo[4,5-b]pyridine-6-carboxamide
7- {[2-ethyl-3-({[(1R,2S)-2-hydroxy-2-(4-hydroxyphenyl)-1-methylethyl]amino}methyl)phenyl]amino}-2-(4-morpholin-4-ylphenyl)-3H-imidazo[4,5-b]pyridine-6-carboxamide
7- {[2-ethyl-3-({[(1R,2R)-2-hydroxy-1-(hydroxymethyl)-2-phenylethyl] amino}methyl)phenyl]amino}-2-(4-morpholin-4-ylphenyl)-3H-imidazo[4,5-b]pyridine-6-carboxamide
7-({2-ethyl-3-[(3-hydroxypiperidin-1-yl)methyl]phenyl}amino)-2-(4-morpholin-4-ylphenyl)-3H-imidazo[4,5-b]pyridine-6-carboxamide

7-[(2-ethyl-3-{[(2-hydroxypropyl)amino]methyl}phenyl)amino]-2-pyridin-4-yl-3H-imidazo[4,5-b]pyridine-6-carboxamide
7- [(2-ethyl-3 - {[(2-hydroxy-1-methylethyl)amino]methyl}phenyl)amino]-2-pyridin-4-yl-3H-imidazo[4,5-b]pyridine-6-carboxamide
7-{[2-ethyl-3-(1H-imidazol-1-ylmethyl)phenyl]amino}-2-pyridin-4-yl-3H-imidazo[4,5-b]pyridine-6-carboxamide trifluoroacetate
7-[(3-{[(2,3-dihydroxypropyl)amino]methyl}-2-ethylphenyl)amino]-2-pyridin-4-yl-3H-imidazo[4,5-b]pyridine-6-carboxamide
7-{[2-ethyl-3-(morpholin-4-ylmethyl)phenyl]amino}-2-pyridin-4-yl-3H-imidazo[4,5-b]pyridine-6-carboxamide trifluoroacetate
7-{[3-({[(1S)-2-cyclohexyl-1-(hydroxymethyl)ethyl]amino}methyl)-2-ethylphenyl]amino}-2-pyridin-4-yl-3H-imidazo[4,5-b]pyridine-6-carboxamide
7-[(2-ethyl-3-{[4-(2-hydroxyethyl)piperazin-1-yl]methyl}phenyl)amino]-2-pyridin-4-yl-3H-imidazo[4,5-b]pyridine-6-carboxamide
7-({2-ethyl-3-[(3-hydroxypyrrolidin-1-yl)methyl]phenyl}amino)-2-pyridin-4-yl-3H-imidazo[4,5-b]pyridine-6-carboxamide
7- {[2-ethyl-3-({[(1S)-2-hydroxy-1-phenylethyl]amino}methyl)phenyl]amino}-2-pyridin-4-yl-3H-imidazo[4,5-b]pyridine-6-carboxamide
7- [(2-ethyl-3 - {[(2R)-2-(hydroxymethyl)pyrrolidin-1-yl]methyl}phenyl)amino]-2-pyridin-4-yl-3H-imidazo[4,5-b]pyridine-6-carboxamide
7- {[2-ethyl-3-({[(1S,2S)-2-hydroxy-1-(hydroxymethyl)-2-phenylethyl]amino}methyl)phenyl]amino}-2-pyridin-4-yl-3H-imidazo[4,5-b]pyridine-6-carboxamide
7-[(2-ethyl-3-{[(2-hydroxycyclohexyl)amino]methyl}phenyl)amino]-2-pyridin-4-yl-3H-imidazo[4,5-b]pyridine-6-carboxamide
7-({2-ethyl-3-[(3-hydroxypiperidin-1-yl)methyl]phenyl}amino)-2-pyridin-4-yl-3H-imidazo[4,5-b]pyridine-6-carboxamide

### Pharmacological Data

### JAK3 HTRF assay

The JAK3 kinase assay utilizes a fusion protein (Jak3 kinase domain fused to Glutathione S-transferase, GST) coexpressed in E.Coli with GroEL/S, and purified by affinity chromatography on Glutathione Sepharose. The enzyme is diluted in 10 mM Tris-HCl, 150 mM NaCl, 5% mannitol, 2 mM 2-mercaptoetanol and 30% glycerol. The substrate in the kinase reaction is a biotinylated peptide of the autophosphorylation site of JAK3 (biotin-LPDKDYYVVREPG) used at 2 µM. Assay conditions are as follows: JAK3, compound and substrate are incubated in 25 mM Trizma base, 5 mM MgCl₂, 5 mM MnCl2, 0.05% TritonX-100 and 2 µM ATP for 45 min at RT. Reaction volume is 20 µM. Stopsolution is added for a final concentration of 100 µM EDTA. Finally 0.065 mg/ml PT66-K and 10.42 µM SA-XL665 are added in 50 mM Hepes, 0.5 M KF and 0.1% BSA. The plate is read in a Discovery instrument after 60 min incubation.

The compounds of the examples have an IC50 less than 25 µM

## Claims

1. A compound of formula (I): 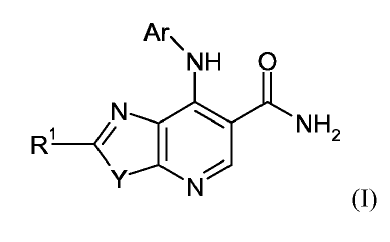 wherein:
Y is NH, S or O;
R¹ is SCH₃, SOCH₃, O-Ph-CONH₂, O(CH₂)₃-N(CH₃)₂, NH(CH₂)₃-morpholine, O(CH₂)₂-morpholine, OCH₃, O(CH₂)₂-N(CH₃)₂, 2-(3-aminophenoxy) or 4-methoxyphenoxy;
R² is a bond, NH, NC₁-C₈ alkyl, S or O;
R³ is NR⁴R⁵ where R⁴ and R⁵ are independently hydrogen or C₁-C₈ alkyl, or R³ is a phenyl ring, a 5 to 7-membered saturated ring containing 1 or 2 heteroatoms selected from nitrogen, oxygen and sulphur, or a 5- to 7-membered heteroaryl group containing 1 to 3 heteroatoms selected from nitrogen oxygen and suphur, each of which can optionally substituted by one or more groups selected from CONR⁴R⁵, NR⁴R⁵, C₁-C₈ alkyl, C₁-C₈ alkoxy, S(O)ₙC₁-C₈ alkyl, hydroxyl, CN, halogen, NHCOC₁-C₈alkyl, R²-(CH₂)ₚ-OH or morpholine;
n is 0, 1 or 2;
p is 0 - 3;
Ar is phenyl which can be optionally substituted by one or more groups selected from halogen, hydroxy, cyano, C₁-C₈ alkyl (itself optionally substituted by one or more hydroxy or cyano groups or fluorine atoms), CH₂-R⁶, CH₂O(CH₂)ₘOC₁₋₆ alkyl, C₁-C₈ alkyl-NR³-R⁴, or a C₂₋₆ alkyl chain optionally containing an NR⁷ group in the chain and optionally substituted by one or more OH groups and optionally terminating in a cycloalkyl or phenyl group, the cycloalkyl and phenyl groups themselves being optionally substituted by one or more hydroxyl groups;
m is 1 to 4;
R⁶ is a 5 to 7-membered saturated ring containing 1 or 2 heteroatoms selected from nitrogen, oxygen and sulphur, an aryl or 5- to 7-membered heteroaryl group containing 1 to 3 heteroatoms selected from nitrogen oxygen and suphur, each of which can optionally substituted by one or more substituents selected from hydroxyl or hydroxymethyl;
R⁷ is hydrogen or C₁₋₆ alkyl;
and pharmaceutically acceptable salts thereof.

2. A compound according to claim 1 in which Y is NH or S.

3. A compound according to claim 1 or 2 in which Ar is phenyl optionally substituted by one or more groups selected from hydroxymethyl, ethyl, [(2-hydroxypropyl)amino]methyl, [(2-hydroxy-1-methylethyl)amino]methyl, (1H-imidazol-1-ylmethyl), [(2,3-dihydroxypropyl)amino]methyl, 3-(morpholin-4-ylmethyl), {[2-cyclohexyl-1-(hydroxymethyl)ethyl]amino}methyl, {[1-benzyl-2-hydroxyethyl]amino}methyl, (3-hydroxypyrrolidin-1-yl)methyl, {[2-hydroxy-1-phenylethyl]amino}methyl, ({2-hydroxy-1-(hydroxymethyl)-2-phenylethyl)amino}methyl, {(2-hydroxycyclohexyl)amino}methyl, {(2-hydroxy-2-(4-hydroxyphenyl)-1-methylethyl]amino}methyl, 3-hydroxypiperidin-1-ylmethyl, {[1-(hydroxymethyl)-2-methylpropyl]amino}methyl, {[4-(methylsulphonyl)benzyl]amino}methyl, or {[2-(3,4-dihydroxyphenyl)-2-hydroxyethyl]amino}methyl.

4. A compound according to claim 1 which is:
7- {[3-(hydroxymethyl)-2-methylphenyl]amino}-2-(methylthio)[1,3]thiazolo[5,4-b]pyridine-6-carboxamide
7-[(2-ethylphenyl)amino]-2-(methylsulfinyl)[1,3]thiazolo[5,4-b]pyridine-6-carboxamide 2-[4-(aminocarbonyl)phenoxy]-7-[(2-ethylphenyl)amino][1,3]thiazolo[5,4-b]pyridine-6-carboxamide
7-[(2-ethylphenyl)amino]-2-[(3-morpholin-4-ylpropyl)amino][1,3]thiazolo[5,4-b]pyridine-6-carboxamide
2-[3-(dimethylamino)propoxy]-7-[(2-ethylphenyl)amino][1,3]thiazolo[5,4-b]pyridine-6-carboxamide
7-[(2-ethylphenyl)amino]-2-(methylthio)[1,3]thiazolo[5,4-b]pyridine-6-carboxamide
7-[(2-ethylphenyl)amino]-2-(2-morpholin-4-ylethoxy)[1,3]thiazolo[5,4-b]pyridine-6-carboxamide
7-[(2-ethylphenyl)amino]-2-methoxy[1,3]thiazolo[5,4-b]pyridine-6-carboxamide
7-[(2-methylphenyl)amino]-2-(methylthio)[1,3]thiazolo[5,4-b]pyridine-6-carboxamide 2-[2-(dimethylamino)ethoxy]-7-[(2-ethylphenyl)amino][1,3]thiazolo[5,4-b]pyridine-6-carboxamide
2-(3-aminophenoxy)-7-[(2-ethylphenyl)amino] [1,3]thiazolo[5,4-b]pyridine-6-carboxamide bis(trifluoroacetate); or
7-[(2-ethylphenyl)amino]-2-(4-methoxyphenoxy)[1,3]thiazolo[5,4-b]pyridine-6-carboxamide; or a pharmaceutically acceptable salt thereof.

5. A compound of formula (I) as defined in any one of claims 1 to 4 for use in therapy

6. A pharmaceutical composition comprising a compound of formula (I) according to claim 1 or a pharmaceutically acceptable salt thereof in association with a pharmaceutically acceptable carrier.

7. A method of treating a disease or condition mediated by JAK3 which comprises administering to a patient in need of such treatment a compound of formula (I) as defined in claims 1 to 4 or a pharmaceuticaly acceptable salt thereof.

8. A method according to claim 7 in which the disease or condition is asthma, host versus graft rejection/transplantation or rheumatoid arthritis.
